# EUROPÄISCHE PATENTSCHRIFT

(11) **EP 1 110 947 B1**
(45) Veröffentlichungstag und Bekanntmachung des Hinweises auf die Patenterteilung: **05.03.2003**
(21) Anmeldenummer: 00128184.9
(22) Anmeldetag: 21.12.2000
(51) Int. Cl.: C07C 277/08, C07C 279/14

(54) **Verfahren zur Reinigung von Kreatin**
Process for the purification of creatine
Procédé pour la purification de creatine

(30) Priorität: 22.12.1999 DE 19962227
(43) Veröffentlichungstag der Anmeldung: 27.06.2001
(73) Patentinhaber: BASF AKTIENGESELLSCHAFT, 67056 Ludwigshafen (DE)
(72) Erfinder: Kessel, Knut, 68161 Mannheim (DE); Scherr, Günter, 67065 Ludwigshafen (DE); Bogenstätter, Thomas, 67098 Bad Dürkheim (DE); Orsten, Stefan, 67158 Ellerstadt (DE); Franke, Dirk, 67067 Ludwigshafen (DE)
(74) Vertreter: Kinzebach, Werner, Dr.

(56) Entgegenhaltungen:
- EP-A- 0 754 679
- US-A- 5 612 375

## Beschreibung

Das Aminosäurederivat Kreatin kommt in der Natur insbesondere als Kreatin-Phosphat im Wirbeltiermuskel vor. Kreatin-Phosphat dient hier als Energieträger der Zelle für muskuläre Kontraktionsenergie. Kreatin findet als Nahrungsergänzung Einsatz bei der Therapie von neuromuskulären Erkrankungen (z.B. Muskeldystrophie) und Endokrinopathien, bei denen es zu einer mangelhaften Kreatinspeicherung und vermehrter -Ausscheidung über den Urin kommt. Neben der Verwendung in Kulturmedien und als Geschmacksverstärker in Gewürzen wird Kreatin in zunehmendem Maße als Nahrungsergänzungsstoff im Sport zur Steigerung der körperlichen Leistungsfähigkeit und insbesondere beim Body-Building eingesetzt.

Das durch chemische Synthese hergestellte oder aus natürlichen Quellen isolierte Kreatin weist in der Regel Verunreinigungen auf, die aus dem Isolierungs- und dem Syntheseprozess oder aus den Edukten der Kreatin-Synthese stammen können. Angesichts der hohen täglichen Verzehrmengen von bis zu 30 g Kreatin z.B. durch Leistungssportler sind an die Reinheit des verzehrten Kreatins hohe Anforderungen zu stellen, um eine Kumulation von Verunreinigungen auszuschließen. Es besteht daher ein Bedürfnis nach Verfahren, die es gestatten, Kreatin auf einfache Weise in höchstmöglicher Reinheit zur Verfügung zu stellen. Außerdem fällt bei den bekannten Herstellungsverfahren das Kreatin meist in einer für Weiterverarbeiter und Endverbraucher unpraktischen kleinen Teilchengröße an. Ein gröberes Produkt wäre deshalb wünschenswert, da dieses weniger staubt und weniger zu elektrostatischer Aufladung und Verbackung neigt.

Die EP 0 754 679 beschreibt ein Verfahren zur Herstellung von Kreatin, bei dem man Cyanamid mit Natrium- oder Kaliumsarkosinat in Wasser bei einer Temperatur von 20 bis 150°C und einem pH-Wert von 7,0 bis 14,0 umsetzt. Das entstehende Kreatin fällt als schwerlösliches Kreatin-Monohydrat aus der wässrigen Lösung aus, welches abgetrennt und anschließend zur Reinigung mit kaltem oder heißem Wasser gewaschen wird. Außerdem wird auf die Möglichkeit einer anschließenden Umkristallisation hingewiesen.

Die JP 59-000 500 beschreibt die Kreatinsynthese durch kontinuierliches Einbringen von O-Alkylisoharnstoff in eine wässrige Sarkosinlösung bei konstant gehaltenem pH-Wert von 10 bis 12 und einer Temperatur von 5 bis 25°C. Die entstehenden Kreatin-Monohydrat-Kristalle werden durch eine Sequenz aus zwei Waschoperationen mit Wasser und Methanol gereinigt. Nachteiligerweise kommt es bei der Synthese zur spontanen Kristallkeimbildung und dadurch zu einer hohen Variabilität der Kristallgröße und zu einem unerwünscht hohen Feingutanteil.

Die DE 197 48 696 beschreibt ein Verfahren zur Herstellung von Kreatin durch 0-Methylierung von Harnstoff und Umsetzung des erhaltenen O-Methylisoharnstoff-salzes mit Natriumsarkosinat. Die erhaltenen Kreatin-Monohydrat-Kristalle werden mit Wasser oder Eiswasser gewaschen.

Beim Herstellungsverfahren der DE-A-198 60 048.8 werden eine wässrige Lösung von O-Alkylisoharnstoff und eine wässrige Lösung von Sarkosinat in Gegenwart vorgelegter Kreatin-Monohydrat-Kristalle umgesetzt. Die entstehenden Kreatin-Monohydrat-Kristalle werden auch hier mit kaltem oder warmem Wasser gewaschen.

Die diesen Herstellungsverfahren gemeinsamen anschließenden Waschoperationen besitzen den Nachteil, dass nur oberflächlich anhaftende Verunreinigungen beseitigt werden, während die auf molekularer Ebene eingebauten bzw. in Form von Tröpfchen der Mutterlauge in den Kristallen eingeschlossenen Verunreinigungen weitgehend unbeeinflußt zurückbleiben.

Eine Umkristallisation bietet zwar die Möglichkeit, einen höheren Reinheitsgrad zu erlangen und darüber hinaus die Kristallgröße genauer einzustellen. Dabei wird die zu reinigende Substanz unter Erwärmen in einem Lösungsmittel, z.B. Wasser, gelöst und kristallisiert beim Abkühlen in reinerer Form aus der Lösung aus. Die nach dem Abtrennen der Kristalle erhaltene gesättigte Lösung, in der die gelösten Verunreinigungen zurückbleiben, wird im Allgemeinen als "Mutterlauge" bezeichnet. Allerdings ist die Umkristallisation von Kreatin aus Wasser insofern schwierig, als sowohl die absolute Löslichkeit als auch die Temperaturabhängigkeit der Löslichkeit von Kreatin in Wasser gering sind. Die Löslichkeit des Kreatins in Wasser beträgt beispielsweise lediglich etwa 1% bei 0°C und etwa 9% bei 80°C. Ferner kann die Temperaturdifferenz zwischen dem Löse- und Kristallisationsvorgang nicht beliebig erhöht werden, denn oberhalb einer Temperatur von etwa 80°C nimmt die Zersetzung des Kreatins zu Zersetzungsprodukten, wie Kreatinin und N-Methylhydantoin, in nicht mehr akzeptablem Maße zu. Im Ergebnis sind zur Umkristallisation von Kreatin aus Wasser im Allgemeinen mindestens 12 Gewichtsteile Wasser pro Gewichtsteil Kreatin erforderlich. Es fällt daher ein großes Volumen Mutterlauge an, das aufgearbeitet oder entsorgt werden muß. Für den Erhalt von größeren Kristallen ist bei der Umkristallisation typischerweise eine langsame Abkühlung erforderlich.

Die Umkristallisation aus anderen Lösungsmitteln als Wasser, in denen Kreatin besser löslich und die Temperaturabhängigkeit der Löslichkeit stärker ausgeprägt ist, ist in der Literatur nicht beschrieben. Bei Verwendung organischer Lösungsmittel würde sich darüber hinaus das Problem der rückstandsfreien Entfernung des Lösungsmittels ergeben.

Der vorliegenden Erfindung liegt daher die Aufgabe zugrunde, ein Verfahren zur Reinigung von Kreatin anzugeben, das die genannten Nachteile nicht aufweist.

Überraschenderweise wurde ein großtechnisch wirtschaftlich durchführbares Reinigungsverfahren gefunden, das gleichzeitig auch eine gezielte Beeinflussung der Kristallgröße zuläßt, bei dem man die thermodynamisch begünstigte Umwandlung von kristallwasserfreiem Kreatin zu Kreatin-Monohydrat ausnutzt.

Gegenstand der Erfindung ist daher ein Verfahren zur Reinigung von Kreatin, wobei das zu reinigende Kreatin (im Folgenden auch "Roh-Kreatin") in kristallwasserfreier Form in eine gesättigte wässrige Lösung von Kreatin-Monohydrat eingetragen wird, wobei das kristallwasserfreie Kreatin unter Hydratisierung gelöst wird, sich Kristalle von Kreatin-Monohydrat aus der wässrigen Lösung abscheiden und diese von der wässrigen Lösung abgetrennt werden.

Unter kristallwasserfreiem Kreatin wird vorliegend eine beliebige Form von Kreatin verstanden, die gegenüber Kreatin-Monohydrat kristallwasserdefizient ist, insbesondere Formen, die pro Mol Kreatin weniger als 0,3 Mol, vorzugsweise weniger als 0,05 Mol Kristallwasser enthalten.

Das Roh-Kreatin hat - ohne Berücksichtigung des Gehaltes an Kristallwasser - in der Regel eine Reinheit von über 95%, bevorzugt über 98%, z.B. bis zu 99,5%. Die Differenz auf 100% wird von Verunreinigungen gebildet, die anorganischer Natur, wie Salze, z.B. Natriumchlorid oder Natriumsulfat, oder organischer Natur, wie Harnstoff, Dicyandiamid, Kreatinin, Guanidin, N-Methylhydantoin, N-Methylhydantoinsäure, Methyliminodiessigsäure, Sarkosin oder Salze davon, sein können. Die Verunreinigungen sind in der Regel wasserlöslich. Das kristallwasserfreie Roh-Kreatin wird vorzugsweise in fein zerteilter Form, z.B. mit einer durchschnittlichen Teilchengröße von weniger als 10 µm, insbesondere weniger als 5 µm, eingesetzt, die gegebenenfalls agglomeriert vorliegen. Die kleine Teilchengröße gewährleistet eine vollständige Auflösung der Teilchen. Bei größeren Teilchen besteht unter Umständen die Gefahr, dass sich rasch eine Haut aus schwerlöslichem Kreatin-Monohydrat bildet, die die weitere Auflösung der Teilchen verzögert oder verhindert.

Das kristallwasserfreie Roh-Kreatin wird im Allgemeinen aus dem beispielsweise als Produkt einer chemischen Synthese primär erhaltenen Kreatin-Monohydrat (Roh-Kreatin-Monohydrat) durch Trocknung z.B. in einem Hordentrockner, Bandtrockner oder durch pneumatische Förderung im Heißluftstrom bei Temperaturen von vorzugsweise bis zu 80°C erhalten. Die Trocknung kann bei Normaldruck oder im Vakuum erfolgen. Bei der Trocknung entweicht das Kristallwasser aus den Schichten der Kristallstruktur des Kreatin-Monohydrats, die Kristalle entwickeln dabei parallel zu den Schichten Risse und zerfallen letztlich vollständig zu einem amorphen Pulver (H. Mendel, D.C. Hodgkin, Acta Cryst. 7, 1954, 443-6; C.S. Frampton, C.C. Wilson, N. Shankland, A.J. Florence, J. Chem. Soc., Faraday Trans. 93(10), 1997, 1875-9).

Eine weitere Möglichkeit der Überführung von Roh-Kreatin-Monohydrat in kristallwasserfreies Roh-Kreatin besteht darin, das Roh-Kreatin-Monohydrat mit einem Lösungsmittel zu versetzen, das mit Wasser ein Azeotrop bildet, wie Ethanol oder Toluol, und das Kristallwasser durch azeotrope Destillation auszukreisen.

Das Roh-Kreatin wird technisch im Allgemeinen durch Guanylierung von Sarkosin, d.h. Übertragung des Guanylrestes (Carbamimidoylrestes) auf Sarkosin oder dessen Salze hergestellt. Als Guanylierungsmittel kommen O-Alkylisoharnstoffsalze, insbesondere O-Methylisoharnstoff-methylsulfat, (vgl. JP 59-000 500, DE 197 48 696 oder DE-A 198 60 048.8) oder Cyanamid (vgl. EP 0 754 679) in Betracht. Dabei werden in der Regel handelsübliche Chemikalien technischer Reinheit eingesetzt, z.B. technische Lösungen von Kalium- oder Natriumsarkosinat mit einer Reinheit von 85 bis 90 Gew.-%. Technische Cyanamidlösungen enthalten in der Regel etwa 5% Dicyandiamid. Die in diesen Chemikalien enthaltenen Verunreinigungen finden sich zum Teil im Roh-Kreatin wieder.

In wässriger Lösung liegt das Kreatinmolekül assoziiert mit einer Hydrathülle aus einer wechselnden Zahl von Wassermolekülen vor. Bei der Kristallisation aus wässriger Lösung kristallisiert Kreatin mit einem Mol Kristallwasser, d.h. als Kreatin-Monohydrat. Mit dem vorliegend verwendeten Begriff "gesättigte wässrige Lösung von Kreatin-Monohydrat" ist eine wässrige Lösung gemeint, die bezüglich Kreatin-Monohydrat gesättigt ist und in der hydratisierte Kreatinmoleküle vorliegen. Die wässrige Lösung kann als Lösungsmittel, lediglich Wasser oder ein Gemisch von Wasser mit z.B. bis zu 70 Gew.-%, vorzugsweise bis zu 30 Gew.-%, wassermischbaren organischen Lösungsmitteln, wie C₁-C₄-Alkanolen, insbesondere Ethanol, enthalten. Die gesättigte wässrige Lösung von Kreatin-Monohydrat kann vorgelegt oder in situ hergestellt werden, indem man kristallwasserfreies Roh-Kreatin in Wasser oder ein Wasser/Lösungsmittel-Gemisch einträgt, wobei sich zunächst eine Menge des Kreatins bis zum Erreichen der Sättigungskonzentration löst. Die Zugabe weiteren kristallwasserfreien Roh-Kreatins führt dann zur Bildung von Kreatin-Monohydrat-Kristallen.

Das Gewichtsverhältnis von wasserfreiem Roh-Kreatin zu gesättigter wässriger Kreatin-Monohydrat-Lösung (beziehungsweise Wasser oder Wasser/Lösungsmittel-Gemisch, aus dem sich in situ durch teilweise Auflösung von Kreatin eine gesättigte Lösung von Kreatin-Monohydrat bildet) ist in weiten Grenzen variierbar und wird nach oben lediglich dadurch begrenzt, dass die sich bildende Suspension von Kreatin-Monohydrat-Kristallen noch eine rührbare Konsistenz aufweist. Es beträgt im Allgemeinen 1:15 bis 1:2, vorzugsweise 1:5 bis 1:2,5. Bei kontinuierlicher Verfahrensführung gelten die angegebenen Verhältnisse für die pro Zeiteinheit zugeführten Mengen an kristallwasserfreiem Roh-Kreatin und Wasser oder Wasser/Lösungsmittel-Gemisch. Damit ist beim erfindungsgemäßen Verfahren ein erheblich geringerer Wasser- bzw. Lösungsmitteleinsatz erforderlich als bei der Umkristallisation. Es fällt demzufolge außerdem eine geringere Menge Mutterlauge an.

Beim erfindungsgemäßen Verfahren finden parallel eine Auflösung des kristallwasserfreien Roh-Kreatins in einer wässrigen Phase und eine Abscheidung kristallinen Kreatin-Monohydrats aus der wässrigen Phase statt. Das Verfahren beruht auf (1) der vergleichsweise hohen Wasserlöslichkeit von wasserfreiem Kreatin, (2) der bereitwilligen Hydratisierung von kristallwasserfreiem Kreatin unter Ausbildung einer Hydrathülle und (3) der vergleichsweise geringen Wasserlöslichkeit von Kreatin-Monohydrat. Die im Roh-Kreatin enthaltenen Verunreinigungen werden bei der Auflösung des kristallwasserfreien Roh-Kreatins verdünnt. Die Verunreinigungen liegen in geringer Konzentration vor und sind im Allgemeinen besser wasserlöslich als Kreatin-Monohydrat, so dass sie überwiegend in der wässrigen Lösung verbleiben. Ist eine höchstmögliche Reinheit angestrebt, wird beim erfindungsgemäßen Verfahren vorzugsweise ein hohes Gewichtsverhältnis von Wasser oder Wasser/Lösungsmittel-Gemisch zu eingetragenem kristallwasserfreien Roh-Kreatin gewählt.

In Gegenwart von Wasser ist Kreatin-Monohydrat gegenüber der kristallwasserfreien Form die thermodynamisch günstigere Form; die negative Freie Enthalpie der Hydratisierung des kristallwasserfreien Kreatins stellt die Triebkraft des erfindungsgemäßen Verfahrens dar. Bei fein zerteiltem kristallwasserfreien Roh-Kreatin ist die Umwandlung zu kristallinem Kreatin-Monohydrat mit einer Verringerung der spezifischen Oberfläche verbunden. Die dadurch bedingte Verringerung der Oberflächenenergie leistet einen zusätzlichen Beitrag zur Triebkraft der Umwandlung. Aufgrund der geringeren Löslichkeit von Kreatin-Monohydrat werden nach Erreichen der Sättigungskonzentration, parallel zum Eintrag von kristallwasserfreiem Roh-Kreatin, Kreatin-Monohydrat-Kristalle abgeschieden.

Vielfach setzt die Kristallisation von Kreatin-Monohydrat nach Überschreiten der Sättigungskonzentration nicht spontan ein. Erst oberhalb einer bestimmten Übersättigung erfolgt eine spontane Keimbildung und Abscheidung von Kreatin-Monohydrat-Kristallen. Diese Vorgänge werden als ungeleitete Kristallisationsprozesse bezeichnet. Ungeleitete Kristallisationsprozesse sind insofern nachteilig, als bei der starken und raschen Kristallkeimbildung Einschlüsse von Mutterlauge in den Kristallen möglich sind und die Kristallgröße nicht gut gesteuert werden kann. Zur Vermeidung dieser Nachteile ist es bevorzugt, das kristallwasserfreie Roh-Kreatin in eine Suspension von Kreatin-Monohydrat-Impfkristallen einzutragen. Durch die Gegenwart der Impfkristalle ist die Verzögerung der Kristallisation aufgehoben. Bei diskontinuierlicher Verfahrensführung werden zweckmäßigerweise Kreatin-Monohydrat-Impfkristalle in einer gesättigten wässrigen Lösung von Kreatin-Monohydrat suspendiert, und das kristallwasserfreie Roh-Kreatin wird in die Suspension eingetragen. Bei kontinuierlicher Verfahrensführung wird das Verfahren zweckmäßigerweise so gesteuert, dass stets ein Teil der bereits gebildeten Kristalle von Kreatin-Monohydrat im Reaktor belassen wird und als Impfkristalle für das Aufwachsen weiteren Kreatin-Monohydrats dient.

Das Inkontaktbringen des kristallwasserfreien Roh-Kreatins mit der gesättigten wässrigen Lösung von Kreatin-Monohydrat erfolgt vorzugsweise unter Bewegung, z.B. durch Rühren mittels geeigneter Rührorgane.

Das Eintragen des kristallwasserfreien Roh-Kreatins und/oder das Austragen der Kreatin-Monohydrat-Kristalle können wahlweise kontinuierlich oder diskontinuierlich erfolgen. In einer besonders bevorzugten Ausführungsform des Verfahrens wird eine gesättigte wässrige Lösung von Kreatin-Monohydrat in einem Reaktor vorgelegt, in den kontinuierlich parallel Wasser oder ein Gemisch von Wasser mit einem wassermischbaren organischen Lösungsmittel und kristallwasserfreies Roh-Kreatin in einem konstanten Verhältnis eingetragen werden und kontinuierlich eine Suspension von Kreatin-Monohydrat-Kristallen ausgetragen wird.

Das Verfahren kann beispielsweise in einem Schlaufenreaktor, Rührkessel oder in einer Kesselkaskade ausgeführt werden.

Bevorzugt verwendet man eine zwei- oder mehrstufige Anordnung mit wenigstens einem Löse- und Hydratisierreaktor, in dem das kristallwasserfreie Roh-Kreatin mit der wässrigen Suspension von Kreatin-Monohydrat-Kristallen in Kontakt gebracht wird, und wenigstens einem Nachkristallisierreaktor, in welchem die Kristalle weiterwachsen und ausreifen. Der Produktstrom vom Löse- und Hydratisierreaktor zum Nachkristallisierreaktor kann periodisch oder kontinuierlich erfolgen, wobei vorzugsweise durch geeignete Maßnahmen verhindert wird, dass ungelöstes kristallwasserfreies Roh-Kreatin in den Nachkristallisierreaktor gelangt. Aus dem Nachkristallisierreaktor wird kontinuierlich oder periodisch eine Suspension von Kreatin-Monohydrat-Kristallen abgezogen, aus der die Kristalle, z.B. durch Filtration oder Zentrifugation, abgetrennt werden. Die nach Abtrennung der Kristalle zurückbleibende Mutterlauge enthält die mit dem Roh-Kreatin eingeführten Verunreinigungen; sie wird aus dem Verfahren ausgeleitet, um eine Anhäufung der Verunreinigungen im Verfahren zu vermeiden. Das mit der ausgeleiteten Mutterlauge entfernte Wasser oder Wasser/Lösungsmittel-Gemisch wird durch kontinuierliche oder periodische Zugaben von Wasser oder Wasser/Lösungsmittel-Gemisch in den Löseund Hydratisierreaktor ersetzt.

Der Löse- und Hydratisierschritt des erfindungsgemäßen Verfahrens wird vorzugsweise bei einer Temperatur von 25 bis 75°C, insbesondere 40 bis 60°C, besonders bevorzugt etwa 50°C durchgeführt. Höhere Temperaturen beschleunigen zwar die Auflösung und Hydratisierung des kristallwasserfreien Roh-Kreatins, führen aber zur partiellen Zersetzung des Kreatins zu Kreatinin und wegen der höheren Löslichkeit von Kreatin-Monohydrat bei der höheren Temperatur zu Ausbeuteverlusten.

Bei Verwendung eines Nachkristallisierreaktors wird in diesem vorzugsweise eine tiefere Temperatur als im Löse- und Hydratisierreaktor eingestellt, um eine vollständigere Abscheidung des gelösten Kreatin-Monohydrats zu erreichen. Im Nachkristallisierreaktor kann z.B. geeigneterweise eine Temperatur von 5 bis 20°C eingestellt werden.

Die stromabwärts gelegene weitere Behandlung der aus dem Reaktor abgezogenen Suspension von Kreatin-Monohydrat-Kristallen, insbesondere die Abtrennung der Kreatin-Monohydrat-Kristalle von der gesättigten wässrigen Lösung von Kreatin-Monohydrat, erfolgt vorzugsweise bei einer Temperatur, die nicht niedriger ist als die Temperatur im Löse- und Hydratisierreaktor beziehungsweise im Nachkristallisierreaktor, falls ein solcher verwendet wird, um das Nachfallen von Kristallen beim Kontakt mit Behälterwänden oder Filterorganen etc. zu vermeiden.

Durch die Wahl der Temperatur und Verweildauer kann die Kristallgröße des nach dem erfindungsgemäßen Verfahren erhaltenen Kreatin-Monohydrats in weiten Grenzen gesteuert werden. Bei höherer Temperatur werden im Allgemeinen größere Kristalle erhalten. Vorzugsweise wird eine mittlere Kristallgröße von 200 bis 600 µm, vorzugsweise 250 bis 500 µm eingestellt.

Die abgetrennten Kristalle von Kreatin-Monohydrat können zur Entfernung anhaftender Reste von Mutterlauge mit kaltem oder warmem Wasser und/oder wassermischbaren organischen Lösungsmitteln gewaschen werden. Sie können dann auf übliche Weise getrocknet werden.

Die nach Abtrennung der Kreatin-Monohydrat-Kristalle erhaltene, die Verunreinigungen des Roh-Kreatins enthaltende gesättigte wässrige Lösung von Kreatin-Monohydrat (Mutterlauge) kann mit Vorteil als wässriges Reaktionsmedium für die Guanylierung von Sarkosin verwendet werden, z.B. um die Edukte beim Syntheseprozess des zu reinigenden Kreatins vorzuverdünnen. Sie kann außerdem als Waschflüssigkeit verwendet werden, um das beim Syntheseprozess erhaltene Roh-Kreatin-Monohydrat zu waschen. Durch diese Verwendungen können die Materialverluste an Wasser, Lösungsmittel, falls ein solches mitverwendet wird, und Kreatin gering gehalten werden. Wenn die gesättigte wässrige Lösung einen hohen Anteil an wassermischbaren organischen Lösungsmitteln enthält, wird die Mutterlauge vorzugsweise nicht als Reaktionsmedium für die Guanylierung verwendet, sondern lediglich zum Waschen des beim Syntheseprozess erhaltenen Roh-Kreatin-Monohydrats.

Die vorliegende Erfindung betrifft demgemäß auch ein Verfahren zur Herstellung von Kreatin-Monohydrat, bei dem man
a) Sarkosin in einem wässrigen Medium mit einem Guanylierungsmittel umsetzt, wobei sich rohes Kreatin-Monohydrat abscheidet,
b) das rohe Kreatin-Monohydrat vom wässrigen Medium abtrennt,
c) gegebenenfalls das abgetrennte rohe Kreatin-Monohydrat mit einer Waschflüssigkeit wäscht,
d) das rohe Kreatin-Monohydrat in kristallwasserfreies Kreatin überführt,
e) das kristallwasserfreie Kreatin in eine gesättigte wässrige Lösung von Kreatin-Monohydrat einträgt, wobei das kristallwasserfreie Kreatin unter Hydratisierung gelöst wird und sich Kristalle von Kreatin-Monohydrat abscheiden,
f) die Kristalle von Kreatin-Monohydrat von der wässrigen Lösung abtrennt und die abgetrennte wässrige Lösung zumindest teilweise als wässriges Medium in Schritt a) und/oder als Waschflüssigkeit in Schritt c) verwendet.

Geeignete Guanylierungsmittel sind die oben genannten. Das Sarkosin wird vorzugsweise in Form seines Natrium- oder Kaliumsalzes eingesetzt.

Das erfindungsgemäße Verfahren wird durch die beigefügten Figuren und die folgenden Beispiele näher veranschaulicht.

Fig. 1 zeigt ein Röntgendiffraktogramm von Roh-Kreatin-Monohydrat-Kristallen, die durch Umsetzung von Natriumsarkosinat mit O-Methylisoharnstoff-methylsulfat in wässriger Lösung erhalten wurden.

Fig. 2 zeigt ein Röntgendiffraktogramm von kristallwasserfreiem Roh-Kreatin (etwa 0,5 Gew.-% Wassergehalt).

Fig. 3 zeigt ein Röntgendiffraktogramm von Kreatin-Monohydrat, das nach dem erfindungsgemäßen Verfahren gereinigt wurde.

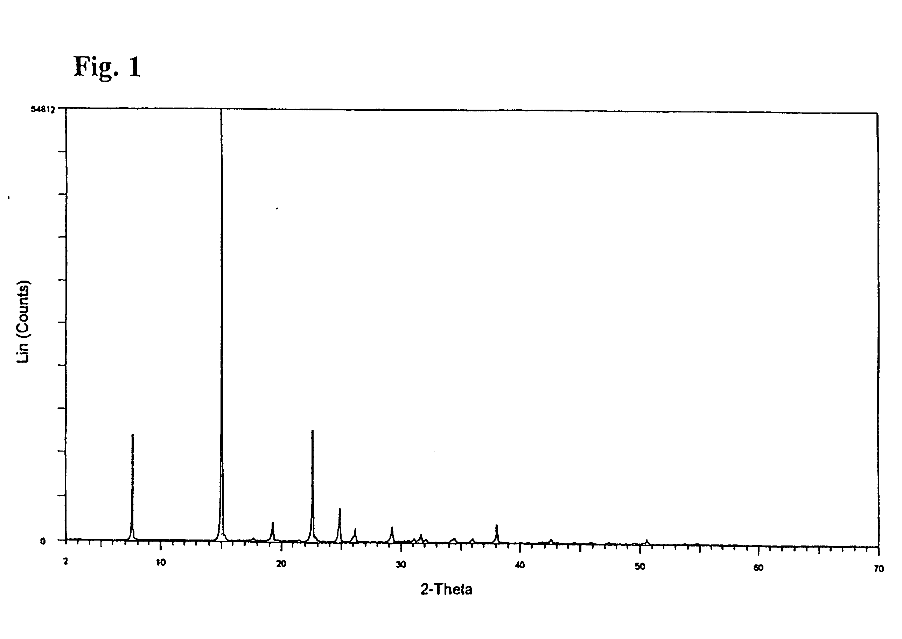

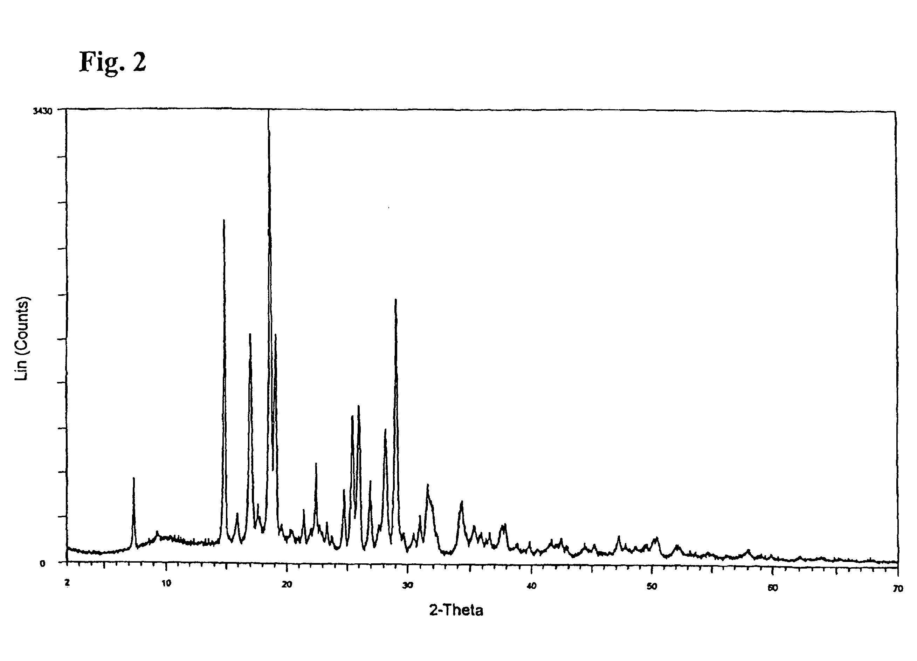

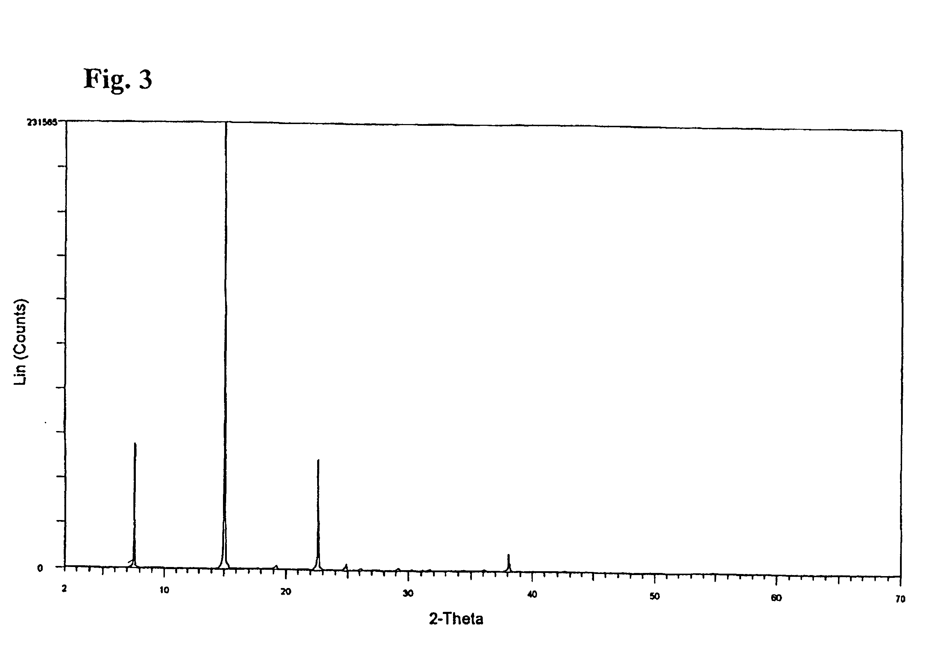

Das Diffraktogramm von Fig. 3 zeigt im Vergleich zum Diffraktogramm von Fig. 1 eine geringere Anzahl von Banden mit höherer Bandenschärfe. Dies deutet auf eine höhere Kristallinität des erfindungsgemäß behandelten Kreatin-Monohydrats hin. Das Diffraktogramm von Fig. 2 zeigt zahlreiche Banden geringer Intensität. Dies weist darauf hin, dass das kristallwasserfreie Roh-Kreatin bis auf eine "Reststruktur" weitgehend amorph ist.

### Beispiel 1

In einem 2000 ml großen doppelwandigen Planschliffkolben mit Rührer wurden 500 ml Trinkwasser auf 50°C erhitzt und bis zur Löslichkeitsgrenze mit festem Kreatin-Monohydrat versetzt. Über einen Dosimaten wurde Trinkwasser in einer Dosiergeschwindigkeit von 1,88 ml/min zugegeben; über eine Dampfsperre wurden zudem 0,70 g/min wasserfreien Kreatins mit einem Gehalt an Dicyandiamid von 330 ppm (Restfeuchte unter 0,1%) in Form von etwa 3 µm großen Teilchen mittels einer Dosierschnecke eingetragen. Sobald der Füllstand ein Volumen von 1500 ml erreichte, floß die etwa 30%-ige warme Suspension von Kreatin-Monohydrat getaktet in einen darunter befindlichen doppelwandigen Planschliffkolben ab, der mit einem Kryostaten auf einer Temperatur von 15°C gehalten wurde. Aus Volumen und Dosierrate ergab sich eine mittlere Verweilzeit von 10 Stunden. Sobald auch in diesem 2000 ml großen Apparat ein Füllstand von 1500 ml erreicht war, lief das Material getaktet auf eine Vakuumsaugnutsche ab, in der eine Metallsinterplatte als Filtrationsorgan diente. Alle 24 h wurde das saugfeuchte Material abgetrennt, einmal mit Trinkwasser gewaschen und in dünner Schicht an der Luft getrocknet. Die Kristallite wiesen einen mittleren Teilchendurchmesser von 480 µm auf und zeigten einen Trocknungsverlust von 12,1%. Über die Laufzeit der Anlage von 2 Wochen näherte sich diese dem stationären Zustand an und die produzierte Menge erreichte etwa den Wert von 0,76 g/min Kreatin-Monohydrat. Der per HPLC nachgewiesene Gehalt an der unerwünschten Verunreinigung Dicyandiamid im gereinigten Kreatin-Monohydrat betrug 30 ppm.

### Beispiel 2

In einem 2000 ml großen doppelwandigen Planschliffkolben wurden 1880 ml Trinkwasser auf 50°C erhitzt und bis zur Löslichkeitsgrenze mit festem Kreatin-Monohydrat versetzt. Über 10 h verteilt wurden nun portionsweise 700 g wasserfreies Kreatin (Restfeuchte unter 0,1%) in Form von etwa 4 µm großen Teilchen eingetragen, die zuvor innig mit 7 g wasserfreien Natriumsulfates vermischt wurden. Natriumsulfate stellt eine potentielle Verunreinigung der Kreatinsynthese dar. Nachdem über Nacht bei Temperatur weitergerührt wurde, wurde die etwa 30%ige Suspension auf eine Vakuumsaugnutsche abgelassen; alsdann wurde einmal mit 50°C warmem Trinkwasser gewaschen und der Rückstand in dünner Schicht an der Luft getrocknet. Die Kristallite wiesen einen Trocknungsverlust von 12,2% auf und enthielten einen ionenchromatographisch bestimmten Restgehalt an Sulfat von 0,05%.

## Patentansprüche

1. Verfahren zur Reinigung von Kreatin, wobei das zu reinigende Kreatin in kristallwasserfreier Form in eine gesättigte wässrige Lösung von Kreatin-Monohydrat eingetragen wird, wobei das kristallwasserfreie Kreatin unter Hydratisierung gelöst wird, sich Kristalle von Kreatin-Monohydrat aus der wässrigen Lösung abscheiden und diese von der wässrigen Lösung abgetrennt werden.

2. Verfahren nach Anspruch 1, wobei die gesättigte wässrige Lösung von Kreatin-Monohydrat in situ hergestellt wird, indem man das kristallwasserfreie Kreatin in Wasser oder ein Gemisch von Wasser mit einem wassermischbaren organischen Lösungsmittel einträgt.

3. Verfahren nach Anspruch 1, wobei das kristallwasserfreie Kreatin in eine wässrige Suspension von Kreatin-Monohydrat-Impfkristallen eingetragen wird.

4. Verfahren nach Anspruch 3, wobei die wässrige Suspension von Kreatin-Monohydrat-Impfkristallen in einem Reaktor vorgelegt wird, in den kontinuierlich parallel Wasser oder ein Gemisch von Wasser mit einem wassermischbaren organischen Lösungsmittel und kristallwasserfreies Kreatin eingetragen werden und kontinuierlich eine Suspension von Kreatin-Monohydrat-Kristallen ausgetragen wird.

5. Verfahren nach einem der vorhergehenden Ansprüche, wobei die Abtrennung der Kreatin-Monohydrat-Kristalle von der wässrigen Lösung durch Filtration oder Zentrifugation erfolgt.

6. Verfahren nach einem der vorhergehenden Ansprüche, wobei die kristallwasserfreie Form des zu reinigenden Kreatins durch Trocknung von Kreatin-Monohydrat erhalten wird.

7. Verfahren nach einem der vorhergehenden Ansprüche, wobei durch Wahl der Temperatur und Verweildauer eine mittlere Kristallgröße des erhaltenen Kreatin-Monohydrats von 200 bis 600 µm eingestellt wird.

8. Verfahren zur Herstellung von Kreatin-Monohydrat, bei dem man
a) Sarkosin in einem wässrigen Medium mit einem Guanylierungsmittel umsetzt, wobei sich rohes Kreatin-Monohydrat abscheidet,
b) das rohe Kreatin-Monohydrat vom wässrigen Medium abtrennt,
c) gegebenenfalls das abgetrennte rohe Kreatin-Monohydrat mit einer Waschflüssigkeit wäscht,
d) das rohe Kreatin-Monohydrat in kristallwasserfreies Kreatin überführt,
e) das kristallwasserfreie Kreatin in eine gesättigte wässrige Lösung von Kreatin-Monohydrat einträgt, wobei das kristallwasserfreie Kreatin unter Hydratisierung gelöst wird und sich Kristalle von Kreatin-Monohydrat abscheiden,
f) die Kristalle von Kreatin-Monohydrat von der wässrigen Lösung abtrennt und die abgetrennte wässrige Lösung zumindest teilweise als wässriges Medium in Schritt a) und/oder als Waschflüssigkeit in Schritt c) verwendet.

## Claims

1. A process for purifying creatine in which the creatine to be purified in the form free of water of crystallization is introduced into a saturated aqueous solution of creatine monohydrate, in which the creatine free of water of crystallization is dissolved with hydration, crystals of creatine monohydrate precipitate from the aqueous solution and these are separated off from the aqueous solution.

2. A process as claimed in claim 1, wherein the saturated aqueous creatine monohydrate solution is prepared in situ by introducing the creatine free of water of crystallization into water or a mixture of water with a water-miscible organic solvent.

3. A process as claimed in claim 1, wherein the creatine free of water of crystallization is introduced into an aqueous suspension of creatine monohydrate seed crystals.

4. A process as claimed in claim 3, wherein the aqueous suspension of creatine monohydrate seed crystals is charged into a reactor into which water, or a mixture of water with a water-miscible organic solvent, and creatine free of water of crystallization are introduced continuously in parallel and a suspension of creatine monohydrate crystals is discharged continuously.

5. A process as claimed in one of the preceding claims, wherein the creatine monohydrate crystals are separated from the aqueous solution by filtration or centrifugation.

6. A process as claimed in one of the preceding claims, wherein the form which is free of water of crystallization of the creatine to be purified is obtained by drying creatine monohydrate.

7. A process as claimed in one of the preceding claims, wherein a mean crystal size of the resultant creatine monohydrate of from 200 to 600 µm is set by selecting the temperature and residence time.

8. A process for preparing creatine monohydrate, in which
a) sarcosine is reacted in an aqueous medium with a guanylating agent, crude creatine monohydrate separating out,
b) the crude creatine monohydrate is separated off from the aqueous medium,
c) the crude creatine monohydrate which is separated off is, if appropriate, washed with a wash liquid,
d) the crude creatine monohydrate is converted into creatine free of water of crystallization,
e) the creatine free of water of crystallization is introduced into a saturated aqueous creatine monohydrate solution, the creatine free of water of crystallization being dissolved with hydration and creatine monohydrate crystals separating out,
f) the creatine monohydrate crystals are separated off from the aqueous solution and the aqueous solution separated off is used at least in part as aqueous medium in step a) and/or as wash liquid in step c).

## Revendications

1. Procédé pour purifier la créatine dans lequel on introduit la créatine à purifier, exempte d'eau de cristallisation, dans une solution aqueuse saturée de monohydrate de la créatine, ce qui provoque la dissolution de la créatine exempte d'eau de cristallisation avec hydratation et la précipitation de cristaux de monohydrate de créatine à partir de la solution aqueuse, et on sépare ces cristaux de la solution aqueuse.

2. Procédé selon la revendication 1, **caractérisé par le fait que** l'on prépare in situ la solution aqueuse saturée de monohydrate de la créatine en introduisant la créatine exempte d'eau de cristallisation dans de l'eau ou dans un mélange d'eau et d'un solvant organique miscible à l'eau.

3. Procédé selon la revendication 1, **caractérisé par le fait que** l'on introduit la créatine exempte d'eau de cristallisation dans une suspension aqueuse de cristaux d'ensemencement de monohydrate de créatine.

4. Procédé selon la revendication 3, dans lequel on place au départ la suspension aqueuse de cristaux d'ensemencement de monohydrate de créatine dans un réacteur dans lequel on introduit en continu, parallèlement, de l'eau ou, un mélange d'eau et d'un solvant organique miscible à l'eau et de la créatine exempte d'eau de cristallisation et on évacue en continu une suspension de cristaux de monohydrate de créatine.

5. Procédé selon l'une des revendications qui précèdent, dans lequel les cristaux de monohydrate de créatine sont séparés de la solution aqueuse par filtration ou centrifugation.

6. Procédé selon l'une des revendications qui précèdent, dans lequel la forme exempte d'eau de cristallisation de la créatine à purifier est obtenue par séchage du monohydrate de créatine.

7. Procédé selon l'une des revendications qui précèdent, **caractérisé par le fait que**, en agissant sur la température et la durée de passage, on règle le monohydrate de créatine obtenu à une dimension moyenne de cristal de 200 à 600 µm.

8. Procédé pour la préparation du monohydrate de créatine dans lequel
a) on fait réagir la sarcosine dans un milieu aqueux avec un réactif guanylant, ce qui provoque la précipitation du monohydrate de créatine brut,
b) on sépare le monohydrate de créatine brut du milieu aqueux,
c) le cas échéant, on lave le monohydrate de créatine brut séparé à l'aide d'un liquide
d) on convertit le monohydrate de créatine brut en créatine exempte d'eau de cristallisation,
e) on introduit la créatine exempte d'eau de cristallisation dans une solution aqueuse saturée de monohydrate de la créatine, ce qui provoque la dissolution de la créatine exempte d'eau de cristallisation avec hydratation et la précipitation de cristaux de monohydrate de créatine,
f) on sépare les cristaux de monohydrate de créatine de la solution aqueuse et on utilise la solution aqueuse séparée, en partie au moins, en tant que milieu aqueux au stade a) et/ou en tant que liquide de lavage au stade c).
